Europäisches Patentamt

European Patent Office

Office européen des brevets

(11) Publication number: **0 103 870**
**B1**

# EUROPEAN PATENT SPECIFICATION

(45) Date of publication of patent specification: **25.11.87**

(21) Application number: **83109201.0**

(22) Date of filing: **16.09.83**

(51) Int. Cl.⁴: **C 07 C 41/06,** C 07 C 9/16,
C 07 C 29/80

(54) Combined ether and alkylate production.

(30) Priority: **20.09.82 US 420435**

(43) Date of publication of application:
**28.03.84 Bulletin 84/13**

(45) Publication of the grant of the patent:
**25.11.87 Bulletin 87/48**

(84) Designated Contracting States:
**AT BE CH DE FR GB IT LI LU NL SE**

(56) References cited:
**US-A-4 218 569**

**ISA TRANSACTIONS, vol. 21, no. 2, 1982, pages
1-13, Research Triangle Park, NC. (US), P.B.
FRANKLAND et al.: "Instrumentation and
control of methanol plants"**

(73) Proprietor: **PHILLIPS PETROLEUM COMPANY
5th and Keeler
Bartlesville Oklahoma 74004 (US)**

(72) Inventor: **Van Pool, Joe
1418 S.E. Prairie Heights Dr.
Bartlesville Oklahoma 74003 (US)**

(74) Representative: **Dost, Wolfgang, Dr.rer.nat.,
Dipl.-Chem. et al
Patent- und Rechtsanwälte Bardehle-
Pagenberg-Dost-Altenburg & Partner Postfach
86 06 20
D-8000 München 86 (DE)**

Courier Press, Leamington Spa, England.

## Description

The present invention relates generally to the processing of alcohol to remove undesirable contaminants therefrom. In one aspect the invention relates to a combined process for the production of ether and alkylate wherein alcohol is processed to remove undesirable contaminants therefrom. In another aspect the invention relates to a system for controlling the removal of undesirable contaminants from alcohol in a combined process for the production of ether and alkylate.

It is known in the art to produce ether by the reaction of an alcohol and a tertiary olefin. It is also known in the art to produce high octane hydrocarbons by an HF alkylation reaction in an olefin and an isoparaffin. Both the ether and the alkylate are valuable motor fuel components.

In ISA Transactions, Vol. 21, No. 2, pp. 1—13, the control and instrumentation of methanol plants is described. In the methanol purification topping followed by refining is mentioned. U.S. Patent 4 218 569 describes processing of etherified light hydrocarbon mixtures for methanol removal. Absorption or extraction with glycol is mentioned.

It is also known in the art to produce methyltertiary butyl ether (MTBE) by reacting isobutylene (in admixture with linear butylenes) and methanol and charging the unreacted butylenes to an alkylation plant in which the butylenes and an isoparaffin such as isobutane are reacted to form high octane gasoline components. An MTBE process is described in U.S. Patent 4 299 999.

Whereas the above discussed technologies have been developed to a rather mature stage, several problems have remained. Thus, the combination process of ether formation and alkylation outlined above has the disadvantage that the olefin stream coming from the ether production may contain materials which are either harmful to the alkylation reaction as such or which tend to be released in the fractionation of the alkylation product in gaseous form, building up pressure and requiring venting, thus removing therewith, for instance, HF and/or propane, which are lost from the operation. An additional disadvantage of the above-described combination process is that the source of liquid methanol and even the method of handling liquid methanol for storage results in the methanol having dissolved therein oxygen and nitrogen, as from the air. Purchased methanol from a typical source can have up to about 88 parts per million by weight of oxygen and about 186 parts per million by weight of nitrogen (at 101.3 kPa (760 mm Hg) and 20°C) contained therein. Other contaminants or inerts that can be present are hydrogen, methane, ethane, ethylene, carbon dioxide, carbon monoxide, fuel gas, and/or materials that give undesirable reactions in the alkylation unit or are not condensed in the alkylation unit depropanizer accumulator.

In the process of manufacturing MTBE, the isobutylene-containing hydrocarbon feedstock also contains other olefins, such as butene-1, cis-butene-2 and trans-butene-2, along with normal butane and isobutane. The feed may also contain propane, propylene, isopentane, normal pentane, and amylenes. The unconverted or unreacted linear butylenes are excellent feed components for the HF alkylation of isobutane to produce high octane gasoline. However, when the hydrocarbon phase from the HF alkylation process is fractionated, light gases such as oxygen, nitrogen, methane, fuel gas, ethane, hydrogen, ethylene, and the like must not be present in the fractionation system, and especially should not be allowed to enter the overhead accumulation of the depropanizing zone, since these light gases (all with lower boiling points than propane) pressure up the system and then must be vented from the system. Venting of these light gases, as from the depropanizer overhead accumulator, causes removal therewith of HF vapor and of vaporous propane. This loss of valuable HF and propane cannot be tolerated.

It is thus an object of the present invention to provide a combined ether and alkylate forming process in which the introduction of impurities into both the ether unit and the alkylation unit is minimized.

Another object of this invention is to provide a combined process for the production of ether and alkylate with reduced risk of losses of materials from the alkylation unit caused by undesirable materials entering the alkylation unit from the ether and/or from a source of alcohol.

A further object of this invention is to provide a combined ether forming and alkylate forming process which renders the ether unit and the alkylation unit even more compatible.

Yet another object of this invention is to provide a combined ether forming and alkylate forming process of increased efficiency of operation.

Other objects, aspects, advantages, details, features and embodiments of this invention will become apparent to those skilled in the art from the following detailed description of the invention, the appended claims and the drawings in which:

Figs. 1A and 1B depict a schematic flow diagram of an embodiment of this invention; and

Fig. 2 is a partial schematic flow diagram of an alternate embodiment of this invention.

The invention resides in a process and a control system as defined in the independent claims. Preferred embodiments are contained in the dependent claims.

To prevent loss of HF and propane in the combined operation of ether or MTBE manufacture and HF alkylation of isobutane with the straight chain butenes recovered from the MTBE manufacturing process, the alcohol reactant, preferably methanol for the manufacture of ether, preferably MTBE, before being charged to the ether forming unit, is processed for the removal of light gases, such as oxygen and nitrogen, e.g., as from air, by the process of my invention which includes subjecting the raw alcohol, preferably methanol, containing undesired at least one of oxygen, nitrogen, methane, fuel gas, ethane, hydrogen, and ethylene to a preliminary stripping operation. By employing the preliminary stripping operation, substantially all the undesired at least one of oxygen, nitrogen, methane, fuel gas, ethane, hydrogen, and ethylene is removed from the alcohol, and a purified alcohol is recovered which can be charged to the ether forming unit, and the recovered straight chain butenes from the ether forming unit can then be charged to the HF alkylation unit without the at least one of oxygen, nitrogen, methane, fuel gas, ethane, hydrogen, and ethylene also referred to as "contaminants" and/or "inerts", pressuring up the depropanizer accumulator of the alkylation unit. By employing the process and apparatus of the present invention, substantially no venting of the depropanizer overhead accumulator is required and the desired savings of HF and propane is realized because of the pretreatment of the raw alcohol, e.g., methanol.

More specifically, the present invention improves a process for the production of both an ether and an alkylate. This process comprises contacting olefins and an alcohol to form a first reaction mixture comprising ether, alcohol and a portion of the olefins. From this first reaction mixture ether is recovered as a first product of the process. In the ether forming unit one or more liquids are accumulated in one or more vessels for later use in the ether forming reaction. An olefin-containing stream is separated from the above-mentioned first reaction mixture. This olefin-containing stream, or at least a portion thereof, is contacted with an isoparaffin in the presence of an HF alkylation catalyst under HF alkylation conditions to form a second reaction mixture comprising alkylate. This alkylate is then recovered from the second reaction mixture as a second product of the combination process. This alkylate is generally recovered as a bottoms stream from a fractionation zone in which at least a portion of the second reaction mixture is fractionated. The fractionation zone is provided with an overhead accumulator in which the cooled and condensed overhead effluent from the fractionation zone is accumulated. In accordance with this invention, a feed stream of a mixture containing alcohol and at least one of oxygen, nitrogen, methane, fuel gas, ethane, hydrogen, and ethylene is distilled in a first distillation zone thereby forming a first distillation overhead containing a first portion of the alcohol and the at least one of oxygen, nitrogen, methane, fuel gas, ethane, hydrogen, as well as a first distillation bottoms containing a second portion of the alcohol. At least a portion of the first distillation overhead containing the alcohol and the at least one of oxygen, nitrogen, methane, fuel gas, ethane, hydrogen, and ethylene is passed through a condensing zone forming a liquid phase and a gas phase in a phase separation zone. The separated liquid alcohol phase at least in part is returned to the first distillation zone, and the gaseous at least one of oxygen, nitrogen, methane, fuel gas, ethane, hydrogen, and ethylene phase is vented to atmosphere, flare, or other suitable disposal. The first distillation bottoms are recovered and supplied for use in the previously mentioned step of contacting olefins and an alcohol to form a first reaction mixture comprising ether, alcohol and a portion of the olefins.

In accordance with a preferred embodiment of the process of the present invention, the alcohol employed is preferably methanol and the tertiary olefin is isobutylene, and the ether produced is preferably MTBE.

The ether forming reaction of the combination process of this invention is known in the art both generally and in many of its details. Reference is made to U.S. Patent No. 3,846,088 in which such an ether production process, particularly for the production of MTBE is described. The present invention is applicable to a variety of ether forming units. Usually the ether forming reaction is carried out by contacting alkyl alcohols having one to three carbon atoms, preferably methanol, with monoolefins having four to six carbon atoms under ether forming conditions in the presence of an ether forming catalyst. Preferably, tertiary olefins having four or five carbon atoms and containing a tertiary carbon atom, such as, for example isobutylene and isoamylenes (2 methyl butene-1,2 methyl butene-2) are used in the ether forming reaction. Typical ether forming catalysts include commercially available "Amberlyst 15", "Dowex 50" and "Nalcite HCR". Ether forming reaction conditions and catalysts are well known in the art and are disclosed, for instance, in U.S. Patent 3,846,088, the disclosure of which is herewith incorporated by reference.

Similarly, the alkylation unit of the combination process of this invention is well known. U.S. Patents Nos. 3,213,157, 3,211,536 and 3,309,882 describe such HF alkylation processes. The disclosures of these patents are also incorporated herewith by reference. In the conventional HF alkylation reaction, liquid isoparaffin and liquid olefin are contacted with liquid HF catalyst to form a reaction mixture. The reaction effluent is phase separated into a lower liquid HF catalyst phase and an upper hydrocarbon phase comprising alkylate, unreacted isoparaffin, and dissolved HF. The olefins useful in HF alkylation reactions, and particularly in the combination process of this invention, are olefins having three to five carbon atoms. The isoparaffins used for the alkylation reaction are generally isoparaffins having four to six carbon atoms, isobutane being particularly preferred.

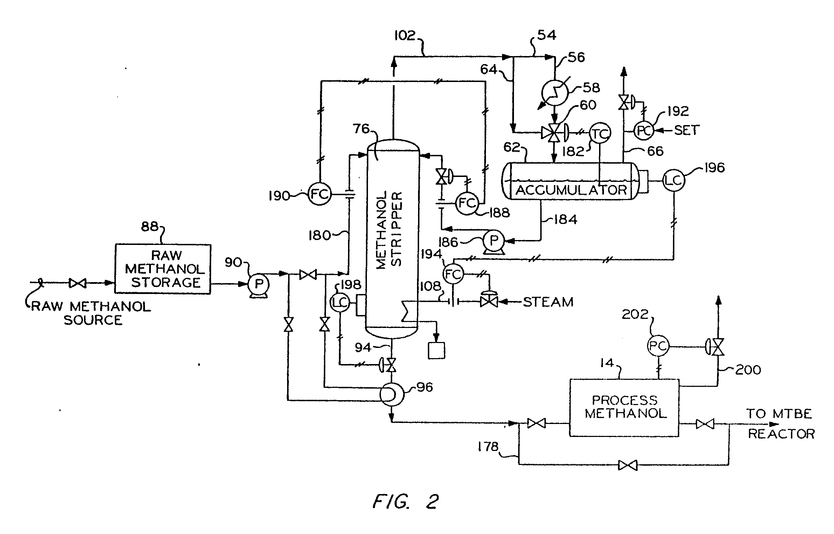

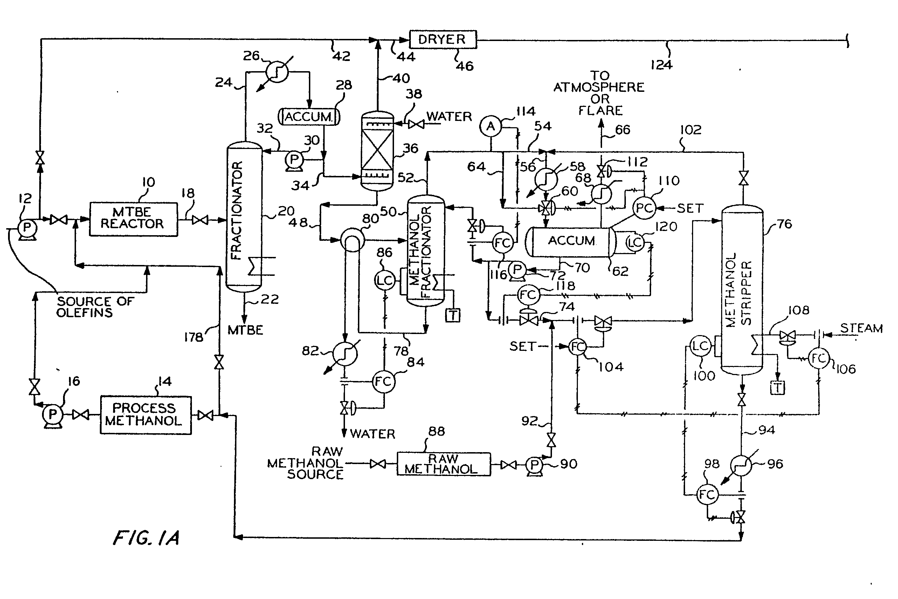

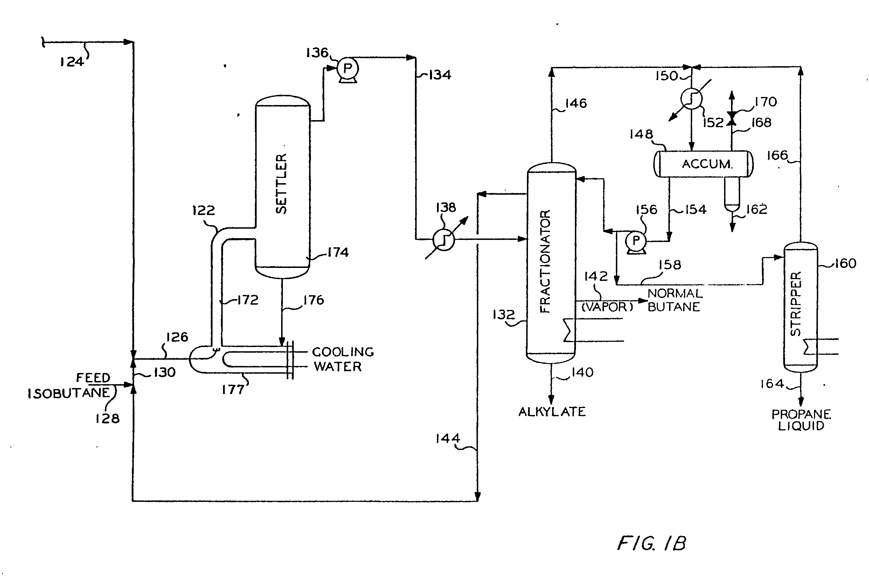

Referring now to the drawings, and to Figs. 1A and 1B in particular, a combined ether and alkylate production process in accordance with the present invention is illustrated in a schematic flow diagram. An ether reactor 10, preferably an MTBE reactor is supplied with a stream comprising olefins, particularly isobutylene, from a suitable source of olefins by means of a pump 12. Process alcohol, preferably methanol, is conveyed to the reactor 10 from an alcohol storage drum 14 by means of a pump 16. The olefins, including at least one tertiary olefin, e.g., isobutylene and methanol are contacted in the reactor 10 under suitable ether reaction conditions to form a reaction mixture which is withdrawn from the reactor 10 via conduit 18. This reaction mixture comprises the ether formed, unreacted olefins and unreacted alcohol. The ether reaction mixture is directed from the conduit 18 into a fractionator 20 wherein the reaction mixture is fractionated and the ether is withdrawn from the fractionator 20 via conduit 22 as a first product of the process. An overhead stream leaves the fractionator 20 via conduit 24 and comprises unreacted

3

olefins and methanol. This gaseous overhead stream is cooled by a suitable indirect heat exchanger 26 and the thereby condensed liquid is accumulated in an accumulator 28. A portion of the accumulated liquid is withdrawn from the accumulator 28 and is reintroduced by means of a pump 30 and conduit 32 into the fractionator 20 as a reflux stream. The remaining or yield portion of the accumulated liquid is passed from the accumulator 28 via conduit 34 to an alcohol washing column 36 into which water is introduced via conduit 38. A stream comprising mainly isobutane and the unreacted olefins and being substantially free of alcohol (in this specific example a stream containing mainly isobutane with linear butylenes as the olefin and substantially free of methanol) leaves the alcohol washing column 36 via conduit 40. This olefin containing stream is combined with another olefin containing stream passing from the pump 12 via conduit 42 (by-pass around MTBE Reactor 10) and this combined stream passes via conduit 44 to a suitable dryer 46 wherein any water contained therein from the alcohol washing column 36 is removed.

Wet alcohol, preferably methanol, leaves the alcohol washing column 36 via conduit 48 and is introduced into distillation means in the form of an alcohol fractionator 50. Alcohol (preferably methanol) containing a small amount of water leaves the alcohol fractionator 50 as an overhead stream via conduit 52. At least a portion of this alcohol containing a small amount of water alcohol is passed as vapor via conduits 54 and 56 to a cooler 58, and the thus condensed alcohol is passed from the cooler 58 via 3-way valve 60 to a separating zone, preferably in the form of an alcohol accumulator 62, where liquid alcohol, in this example methanol, is accumulated. A conduit 64 interconnects conduit 54 and the 3-way valve 60 to provide a bypass for the essentially water free alcohol vapor around the cooler 58 to the accumulator 62. Light gaseous contaminants and/or inerts, for example at least one of oxygen, nitrogen, methane, fuel gas, ethane, hydrogen and ethylene are vented from the accumulator 62 via conduit 66 and a suitable cooler 68 interposed therein allowing venting of contaminants and/or inerts to the atmopshere or to a suitable flare for combustion prior to release to the atmosphere. The cooler 68 functions to condense any alcohol vapor vented from the accumulator 62 to prevent alcohol loss. A portion of the accumulated alcohol in the alcohol accumulator 62 is passed via conduit 70 and pump 72 to the alcohol fractionator 50 as a reflux stream. The remaining or yield portion of the alcohol accumulated in the alcohol accumulator 62 is passed via conduit 70, pump 72 and conduit 74 to a suitable distillation zone preferably in the form of an alcohol stripper column 76. Water is withdrawn from the bottom of the alcohol fractionator 50 via conduit 78 and indirect heat exchangers 80 and 82 interposed in conduit 78 for recovering heat from the water. This water in part can be recycled to conduit 38 by a suitable conduit (not shown) as a portion of the wash water for removing the alcohol from the stream comprising unreacted olefins and alcohol.

Flow of the water through the conduit 78 is preferably controlled by suitable flow control means 84 interposed in the conduit 78 responsive to a suitable level controller 86 which senses the level of liquid water in the lower portion of the alcohol fractionator 50, and regulates the flow of the water through the conduit 78 such that the water level within the alcohol fractionator 50 is maintained within a suitable range.

Raw alcohol, in this example methanol, is received from a suitable source of raw alcohol into a suitable alcohol storage vessel 88.

Raw alcohol from the alcohol storage vessel 88 is conveyed to the alcohol stripper column 76 via a pump 90, a conduit 92 and previously described conduit 74. The raw alcohol from the alcohol storage vessel is combined with the yield portion of the alcohol from the alcohol accumulator 62 at the juncture of the conduits 74 and 92, and the resulting combined alcohol stream is introduced into the alcohol stripper column 76. An alcohol distillation bottoms stream of liquid alcohol is passed from the lower end of the alcohol stripper column 76 to the alcohol storage drum 14 via conduit 94 and a suitable indirect heat exchanger 96 interposed in the conduit 94 to provide cooling of the liquid alcohol and heat recovery. The flow of liquid alcohol through conduit 94 is regulated by suitable flow control means 98 operatively related to a suitable level controller 100 in the lower portion of the alcohol stripper column 76, whereby the flow of liquid alcohol through the conduit 94 is constantly adjusted to maintain the level of liquid alcohol in the lower portion of the column 76 within a predetermined range as sensed by the level controller 100.

A distillation overhead vapor stream containing a portion of the alcohol fed into the alcohol stripper column 76, as well as light gaseous contaminants and/or inerts, such as, for example, at least one of oxygen, nitrogen, methane, fuel gas, ethane, hydrogen, and ethylene is passed from the upper end of the alcohol stripper column 76 via conduit 102 to the juncture of previously described conduits 54 and 56. At the juncture of conduits 54, 56 and 102, the distillation overhead stream from the alcohol stripper column 76 is combined with the alcohol overhead stream from the alcohol fractionator 50 and is conducted through the previously described cooler 58 and 3-way valve 60 into the alcohol accumulator 62. It will be understood that the gaseous inerts introduced into the alcohol accumulator 62 as a consequence of the introduction of the distillation overhead stream from the alcohol stripper column 76 into the alcohol accumulator 62 will be conducted from the accumulator 62 via the conduit 66 and cooler 68 for venting to the atmosphere or to a suitable flare as has been previously described.

Suitable flow control means 104, having a predetermined set point, is interposed in conduit 74 intermediate conduit 92 and the alcohol stripper column 76. The flow control means 104 is operatively connected by suitable means to suitable flow control means 106 interposed in a steam supply line 108 which supplies steam to the lower end of the alcohol stripper column 76 to thereby control the heat input into the alcohol stripper column 76 to thus control the operation of the alcohol stripper column 76 in a predetermined manner.

4

A suitable pressure controller 110 is operatively connected to the alcohol accumulator 62 to monitor the pressure therein. Pressure controller 110 is operatively connected by suitable means to the 3-way valve 60 and a control valve 112 interposed in conduit 66 whereby the valves 60 and 112 are automatically actuated to maintain the pressure in the alcohol accumulator 62 at a predetermined set point.

A suitable analyzer 114 is connected in fluid flow communication with the conduit 52 to monitor the alcohol content of the overhead passing from the alcohol fractionator 50. The analyzer 114 is operatively connected by suitable means to suitable flow control means 116 interposed in conduit 70 intermediate the pump 72 and the alcohol fractionator 50 whereby the flow of the reflux stream to the alcohol fractionator 50 is increased in response to the sensing by the analyzer 114 of the presence of too little alcohol (too much water) in the overhead from the alcohol fractionator 50 to thereby prevent the removal of excessive water in the overhead.

Suitable flow control means 118 are interposed in the conduit 74 intermediate conduits 70 and 92, are operatively connected by suitable means to a level controller 120, operatively related to the alcohol accumulator 62 and responsive to the level of liquid alcohol therein, for controlling the flow of liquid alcohol through the conduit 74 in response to signals from the level controller 120 to thereby maintain the liquid level within the accumulator 62 within a predetermined range.

An olefin feedstream comprising dried olefins, (including isobutane and normal butane) from the dryer 46 is introduced into an alkylation reactor 122 via conduits 124 and 126. Also introduced into the alkylation reactor is an isoparaffin-containing stream, preferably an isobutane-containing stream, from a suitable isoparaffin source via conduits 128, 130 and 126. The liquid hydrocarbon phase is passed from the upper portion of the alkylation reactor to a fractionator 132 via conduit 134, pump 136 and a suitable heater 138 interposed in conduit 134. An alkylate stream is withdrawn from the bottom of the fractionator 132 via conduit 140. A paraffin vapor stream, usually n-butane, is withdrawn from the fractionator 132 via conduit 142. Isobutane liquid is withdrawn from the fractionator 132 via conduit 144 and is recycled to the HF alkylation reactor 122 via conduit 144 and conduits 130 and 126, and the overhead vapor stream comprising propane vapor and HF vapor is withdrawn from the upper end of the fractionator 132 via conduit 146 and is passed to an overhead accumulator 148 via conduit 150 and a suitable cooler-condenser 152 interposed in conduit 150. A portion of the liquid phase in the accumulator 148 is withdrawn via conduit 154 and is passed to the upper end of the fractionator 132 as a reflux stream via pump 156 interposed in conduit 154. The yield portion of the liquid phase is withdarwn from conduit 154 downstream of the pump 156 and is passed via conduit 158 to a stripper 160. Liquid HF is withdrawn from a downwardly extending leg of the overhead accumulator 148 via conduit 162, for recycle to the alkylation reactor by suitable conduit means (not shown). Liquid propane is withdrawn as a bottoms stream from the lower end of the stripper 160 via conduit 164. Propane vapor and HF vapor are passed from the upper end of the stripper 160 as an overhead stream and are recycled to the overhead accumulator 148 via a conduit 166 as well as conduit 150 and cooler-condenser 152. In the event of excess pressure within the overhead accumulator 148, the gaseous phase therein can be vented to the atmosphere or to a suitable flare via conduit 168 and suitable normally closed valve 170 interposed in conduit 168.

The HF alkylation reactor 122 comprises an alkylation reaction zone 172 in which the isobutane feed from stream conduit 126 is alkylated with the olefins from the dryer 46 in the presence of HF catalyst. The HF alkylation reactor 122 further includes a phase separator zone 174 in which the liquid hydrocarbon phase is separated from the liquid HF phase, with the liquid hydrocarbon phase passing from the phase separator zone 174 in the upper portion of the alkylation reactor 122 via the previously mentioned conduit 134, and with the liquid HF phase passing from the bottom of the phase separator zone 174 as a bottoms stream via conduit 176, and the thus separated liquid HF is recycled to the alkylation reaction zone 172 via cooler 177.

It is presently preferred to operate the alcohol stripper column 76 such that from about 5 to about 30 volume percent of the alcohol stream, in this example methanol, preferably about 10 volume percent of the alcohol stream, along with at least one of oxygen, nitrogen, methane, fuel gas, ethane, hydrogen, and ethylene, is taken from the alcohol stripper column 76 as overhead through conduit 102.

A typical distillation column for use as the alcohol stripper column 76 is provided with from about 10 to about 20 bubble trays. Typical operating conditions for the alcohol stripper portion of above-described system are as listed in the following table.

Typical operating condition of the alcohol stripper (20 trays)

| | |
|---|---|
| Pressure, kPa (psia) | |
| Bottom of stripper | 275.6 (40) |
| Top of stripper | 248 (36) |
| Accumulator | 220.5 (32) |
| | |
| Temperatures, °C (°F) | |
| Bottom of stripper | 93 (200) |
| Top of stripper | 88 (190) |
| Accumulator | 32 (90) |
| | |
| Typical methanol product at conduit 94 | |
| Oxygen, ppm wt | nil |
| Nitrogen, ppm wt | nil |
| Other contaminants and/or inerts, ppm wt | nil |

It will be further noted that the system illustrated in Fig. 1A is preferably provided with a conduit 178 which provides means for bypassing purified alcohol, in this case methanol, from the alcohol stripper column 76 and conduit 94 around the alcohol storage drum 14 to be directly introduced into the reactor 10 along with the tertiary olefin-containing feed stream.

Referring now to Fig. 2, there is illustrated an alternate form of alcohol purification system for use in the combined ether and alkylate production system of the present invention. The system illustrated in Fig. 2 directs raw alcohol, in this case methanol, from the alcohol storage vessel 88 to the upper end portion of a distillation zone, preferably in the form of a suitable alcohol stripper column 76, via conduit 180 and a suitable pump 90 interposed therein. The purified liquid alcohol bottoms product is directed from the bottom of the column 76 via conduit 94 and indirect heat exchanger 96 to the alcohol storage drum 14. The indirect heat exchanger 96 provides means for preheating the raw alcohol stream passing through conduit 180 to the column 76. The vaporous overhead from the column 76, comprising alcohol, in this case methanol, and various contaminants and/or inerts, such as at least one of oxygen, nitrogen, methane, fuel gas, ethane, hydrogen, and ethylene, is conveyed from the top of the column 76 via conduits 102, 54 and 56, cooler-condenser 58 and 3-way valve 60 to a separating zone, preferably in the form of an alcohol accumulator 62. Conduit 64 provides vapor bypass fluid communication between conduit 102 and the 3-way valve 60. Bypass of the gaseous overhead from the stripper column 76 is controlled by the 3-way valve 60 in response to a suitable temperature controller 182 which is operatively connected to the valve 60 and controls its operation in response to the temperature sensed within the accumulator 62. The liquid phase within the accumulator 62 is passed therefrom to the upper end portion of the stripper column 76 as a reflux stream via conduit 184 and a suitable pump 186 interposed therein. Preferably all of the liquid phase from the accumulator 62 is used for this reflux stream. The flow rate of the reflux stream through conduit 184 is controlled by suitable operatively connected flow control means 188 and 190 in response to the flow rate of raw alcohol feed to the column 76 through conduit 180. The gaseous phase within the accumulator 62, comprising the various contaminants and/or inerts previously contained within the raw alcohol, for example, oxygen and/or nitrogen, is vented from the accumulator 62 via conduit 66 either to the atmosphere or to a suitable flare. Control of flow through the conduit 66 is provided by a suitable pressure controller 192 comprising control valve means interposed in the conduit 66 and responsive to the pressure within the accumulator 62. Reboil heat is provided to the lower portion of the alcohol stripper column 76 from the source of steam through conduit 108. Reboil heat input to the column 76 is controlled by means of suitable flow control means 194 comprising valve means interposed in conduit 108 and operatively connected to a suitable level control 196 on the accumulator 62 which controls steam flow to the column 76 in response to a liquid level sensed within the accumulator 62.

The flow rate of purified liquid alcohol from the bottom of the alcohol stripper column 76 is controlled by a suitable flow control valve interposed in the conduit 94 and operatively connected to a suitable level controller 198 which senses the liquid level within the lower end portion of the column 76 and controls the liquid flow in response to the liquid level within a predetermined range. The alcohol storage drum 14 is preferably provided with pressure relief means in the form of a vent conduit 200 with suitable valve means interposed therein and operatively connected to a suitable pressure controller 202 which controls actuation of the valve means in response to the pressure sensed within the alcohol storage drum 14 so that the storage drum 14 is vented to the atmosphere or to a suitable flare when the pressure within the drum 14 exceeds a predetermined value.

Typical operating conditions for the system illustrated in Fig. 2 are identical to the operating conditions listed above for the system illustrated in Figs. 1A and 1B.

From the foregoing detailed description, it will be readily apparent that the present invention readily overcomes the previously discussed deficiencies of the prior art and meets all the recited objects of the present invention. Changes may be made in the combination and arrangement of parts or elements as heretofore set forth in the specification and shown in the drawings without departing from the invention as defined in the following claims.

# 0 103 870

**Claims**

1. A combination process of ether production and alkylate production which includes the steps of:

(a) contacting olefins including at least one tertiary olefin and an alcohol to form a first reaction mixture comprising ether, alcohol and a portion of the olefin;

(b) recovering ether from said first reaction mixture as a first product of the combination process;

(c) accumulating a liquid in a vessel for later use in contacting step (a);

(d) separating an olefin-containing stream from said first reaction mixture;

(e) contacting at least a portion of said olefin-containing stream with at least one isoparaffin, in particular an isoparaffin having 4 to 6 carbon atoms per molecule, in the presence of an HF-alkylation catalyst under HF-alkylation conditions to form a second reaction mixture comprising alkylate; and

(f) recovering alkylate from said second reaction mixture as a further product of the process as a bottoms stream from a fractionation zone in which at least a portion of the second reaction mixture is fractionated and which fractionation zone is provided with an overhead accumulator in which cooled, condensed overhead effluent from said fractionation zone is accumulated; characterized by distilling a feed stream of a mixture containing an alcohol and at least one of oxygen, nitrogen, methane, fuel gas, ethane, hydrogen, and ethylene in a first distillation zone thereby forming a first distillation overhead containing a first portion of said alcohol and said at least one of oxygen, nitrogen, methane, fuel gas, ethane, hydrogen, and ethylene and a first distillation bottoms containing a second portion of said alcohol; condensing at least a portion of said first distillation overhead containing said alcohol and said at least one of oxygen, nitrogen, methane, fuel gas, ethane, hydrogen and ethylene; thereafter, separating said first distillation overhead in a separating zone into separated liquid alcohol and gaseous at least one of oxygen, nitrogen, methane, fuel gas, ethane, hydrogen, and ethylene; and recovering said first distillation bottoms and supplying said thus recovered first distillation bottoms for use in the contacting step (a).

2. The process of claim 1 characterized in that at least a portion of said separated liquid alcohol is introduced into said first distillation zone as a portion of said feed stream with said mixture containing an alcohol and at least one of oxygen, nitrogen, methane, fuel gas, ethane, hydrogen, and ethylene.

3. The process of claim 1 or 2 characterized further to include:

separating a liquid containing water and alcohol from said first reaction mixture;

distilling said thus separated liquid containing water and alcohol in a second distillation zone thereby forming a second distillation overhead containing said alcohol and a second distillation bottoms containing said water; and

recovering said second distillation overhead containing said alcohol.

4. The process of claim 3 characterized in that at least a portion of said recovered second distillation overhead is condensed to liquid and at least a portion of the thus condensed liquid is introduced into said second distillation zone as a reflux stream.

5. The process of claim 3 characterized in that at least a portion of said recovered second distillation overhead is condensed to liquid and at least a portion of the thus condensed liquid is introduced into said first distillation zone as a portion of said feed stream with said mixture containing an alcohol and at least one of oxygen, nitrogen, methane, fuel gas, ethane, hydrogen, and ethylene.

6. The process of claim 5 characterized in that at least a portion of the thus condensed liquid is introduced into said second distillation zone as a reflux stream.

7. The process of claim 1 characterized in that at least a portion of said separated liquid alcohol is introduced into said first distillation zone as a reflux stream.

8. The process of claim 1 characterized in that from 5 to 30 volume percent of said feed stream of a mixture containing an alcohol and at least one of oxygen, nitrogen, methane, fuel gas, ethane, hydrogen, and ethylene is formed into said first distillation overhead in said distilling step.

9. The process of any of claims 1 to 8 characterized in that said alcohol is methanol.

10. The process of any of claims 1 to 9 characterized in that said gaseous at least one of oxygen, nitrogen, methane, fuel gas, ethane, hydrogen, and ethylene is combusted and vented to atmosphere.

11. A system for controlling the process of any of claims 1 to 10 characterized by means responsive to the flow of said feed stream into said first distillation zone for controlling the heat input into said first distillation zone; or means responsive to the flow of said feed stream into said first distillation zone for controlling the heat input into said first distillation zone, and means responsive to the level of said first distillation bottoms in said first distillation zone for controlling the output of said first distillation bottoms from said first distillation zone; in particular characterized further to include means responsive to the level of separated liquid alcohol in said separating zone for controlling the output of separated liquid alcohol from said separating zone into said first distillation zone as a portion of said feed stream.

12. A system for controlling the process of any of claims 1 to 10 characterized by means responsive to the level of separated liquid alcohol in said separating zone for controlling heat input into said first distillation zone; or means responsive to the flow of said feed stream into said first distillation zone for controlling the introduction of said reflux stream into said first distillation zone; or means responsive to the level of said first distillation bottoms in said first distillation zone for controlling the output of said first distillation bottoms from said first distillation zone; or means responsive to the temperature within said separating zone for controlling the condensing of at least a portion of said first distillation overhead; or

7

## 0 103 870

means responsive to the pressure within said separating zone for controlling the venting of said gaseous at least one of oxygen, nitrogen, methane, fuel gas, ethane, hydrogen, and ethylene from said separating zone.

**Patentansprüche**

1. Kombinationsverfahren zur Herstellung von Ether und Herstellung von Alkylat mit den Verfahrensschritten:

a) Kontaktieren von mindestens ein tertiäres Olefin enthaltenden Olefinen und einen Alkohol zur Bildung eines ersten Reaktionsgemisches, welches Ether, Alkohol und einen Teil des Olefins enthält;

b) Gewinnung von Ether aus dem ersten Reaktionsgemisch als erstes Produkt des Kombinationsverfahrens;

c) Speichern einer Flüssigkeit in einem Kessel zur späteren Verwendung in dem Kontaktierungsschritt (a);

d) Abtrennen eines Olefin enthaltenden Stromes aus dem ersten Reaktionsgemisch;

e) Kontaktieren zumindest einen Teiles des Olefin enthaltenden Stromes mit zumindest einem Isoparaffin, insbesondere einem Isoparaffin mit 4 bis 6 Kohlenstoffatomen pro Molekül, in Gegenwart eines HF-Alkylierungskatalysators unter HF-Alkylierungsbedingungen zur Bildung eines zweiten, Alkylat enthaltenden Reaktionsgemisches; und

f) Gewinnen von Alkylat aus dem zweiten Reaktionsgemisch als weiteres Verfahrensprodukt als Sumpfproduktstrom aus .einer Fraktionierungszone, in der mindestens ein Teil des zweiten Reaktionsgemisches fraktioniert wird, und wobei die Fraktionierungzone mit einem Kopfproduktakkumulator ausgerüstet ist, in dem gekühlter, kondensierter Kopfproduktabfluß aus der Fraktionierungszone gesammelt wird; dadurch gekennzeichnet, daß man einen Beschickungsstrom eines Gemisches, das einen Alkohol und mindestens einen Vertreter aus Sauerstoff, Stickstoff, Methan, Brenngas, Ethan, Wasserstoff und Ethylene enthält, in einer ersten Destillationszone destilliert unter Bildung eines ersten Destillations-Kopfprodukts, das einen ersten Teil des Alkohols und des zumindest einen Vertreters aus Sauerstoff, Stickstoff, Methan, Brenngas, Ethan, Wasserstoff und Ethylen enthält und eines ersten Destillations-Sumpfproduktes, das einen zweiten Teil des Alkohols enthält; mindestens einen Teil des ersten Destillations-Kopfprodukts, der den Alkohol und den mindestens einen Vertreter aus Sauerstoff, Stickstoff, Methan, Brenngas, Ethan, Wasserstoff und Ethylen enthält, kondensiert; danach das erste Destillations-Kopfprodukt in einer Trennzone in flüssigen Alkohol und gasförmigen zumindest einen Vertreter aus Sauerstoff, Stickstoff, Methan, Brenngas, Ethan, Wasserstoff und Ethylen trennt; und die ersten Destillations-Sumpfprodukte zurückgewinnt und die so zurückgewonnenen ersten Destillations-Sumpfprodukte der Verwendung in dem Kontaktierungsschritt (a) zuführt.

2. Verfahren nach Anspruch 1, dadurch gekennzeichnet, daß man mindestens einen Teil des abgetrennten flüssigen Alkohols in die erste Destillationszone einführt als Teil des Beschickungsstromes mit dem Gemisch, das einen Alkohol und mindestens einen Vertreter aus Sauerstoff, Stickstoff, Methan, Brenngas, Ethan, Wasserstoff und Ethylen enthält.

3. Verfahren nach Anspruch 1 oder 2, dadurch gekennzeichnet, daß es weiter folgendes einschließt: Abtrennen einer Wasser und Alkohol enthaltenden Flüssigkeit von dem ersten Reaktionsgemisch; Destillieren der so abgetrennten, Wasser und Alkohol enthaltenden Flüssigkeit in einer zweiten Destillationszone unter Bildung eines zweiten Destillations-Kopfprodukts, das den Alkohol und ein zweites, das Wasser enthaltende Destillations-Sumpfprodukt enthält; Gewinnen des den Alkohol enthaltenden zweiten Destillations-Kopfproduktes.

4. Verfahren nach Anspruch 3, dadurch gekennzeichnet, daß man mindestens einen Teil des gewonnenen zweiten Destillations-Kopfproduktes zu Flüssigkeit kondensiert und mindestens einen Teil der so kondensierten Flüssigkeit in die zweite Destillationszone als Rückflußstrom einführt.

5. Verfahren nach Anspruch 3, dadurch gekennzeichnet, daß man mindestens einen Teil des gewonnenen zweiten Destillations-Kopfproduktes zu Flüssigkeit kondensiert und mindestens eine Teil der so kondensierten Flüssigkeit in die erste Destillationszone einführt als Teil des Beschickungsstromes mit dem Gemisch, das Alkohol und mindestens einen Vertreter aus Sauerstoff, Stickstoff, Methan, Brenngas, Ethan, Wasserstoff und Ethylen enthält.

6. Verfahren nach Anspruch 5, dadurch gekennzeichnet, daß man mindestens einen Teil des so kondensierten Flüssigkeit in die zweite Destillationszone als Rückflußstrom einführt.

7. Verfahren nach Anspruch 1, dadurch gekennzeichnet, daß man mindestens einen Teil des abgetrennten flüssigen Alkohols in die erste Destillationszone als Rückflußstrom einführt.

8. Verfahren nach Anspruch 1, dadurch gekennzeichnet, daß von 5 bis 30 Volumen % des Beschickungsstromes aus einem Gemisch, daß einen Alkohol und zumindest einen Vertreter aus Sauerstoff, Stickstoff, Methan, Brenngas, Ethan, Wasserstoff und Ethylen enthält, in das erste Destillations-Kopfprodukt in dem Destillationsschritt gebildet wird.

9. Verfahren nach einem der Ansprüche 1 bis 8, dadurch gekennzeichnet, daß der Alkohol Methanol ist.

10. Verfahren nach einem der Ansprüche 1 bis 9, dadurch gekennzeichnet, daß man den gasförmigen zumindest einen Vertreter aus Sauerstoff, Stickstoff, Methan, Brenngas, Ethan, Wasserstoff und Ethylen verbrennt und in die Atmosphäre abläßt.

8

**0 103 870**

11. Steuersystem für das Verfahren nach einem der Ansprüche 1 bis 10, gekennzeichnet durch eine Einrichtung, die auf den Fluß des Beschickungsstromes in die erste Destillationszone zum Regeln des Wärmeeintrags in die erste Destillationszone antwortet; oder eine Einrichtung, die auf den Fluß des Beschickungsstromes in die erste Destillationszone zum Regeln des Wärmeeintrags in die erste Destillationszone antwortet, und einen Einrichtung, die auf den Pegel des ersten Destillations-Sumpfproduktes in der ersten Destillationszone zum Regeln des Austrags des ersten Destillations-Kopfproduktes aus der ersten Dsetillationszone antwortet; insbesondere weiter dadurch gekennzeichnet, daß sie eine Einrichtung einschließt, die auf den Pegel des abgetrennten flüssigen Alkohols in der Trennzone zum Regeln des Austrags des abgetrennten flüssigen Alkohols aus der Trennzone in die erste Destillationszone als Teil des Beschickungsstromes antwortet.

12. Steuersystem für das Verfahren nach einem der Ansprüche 1 bis 10, gekennzeichnet durch eine Einrichtung, die auf den Pegel des abgetrennten flüssigen Alkohols in der Trennzone zum Steuern des Wärmeeintrags in die erste Destillationszone antwortet; oder eine Einrichtung, die auf den Fluß des Beschickungsstromes in die erste Destillationszone zum Regeln der Zuführung des Rückflußstromes in die erste Destillationszone antwortet; oder eine Einrichtung, die auf den Pegel des ersten Destillations-Sumpfproduktes in der ersten Destillationszone zum Regeln des Austrags des ersten Destillations-Sumpfproduktes aus der ersten Destillationszone antwortet; oder eine Einrichtung, die auf die Temperatur in der Trennzone zum Regeln des Kondensierens von mindestens einem Teil des ersten Destillations-Kopfproduktes antwortet; oder eine Einrichtung, die auf den Druck in der Trennzone zum Steuern des Ablassens des gasförmigen zumindest einen Vertreters aus Sauerstoff, Stickstoff, Methan, Brenngas, Ethan, Wasserstoff und Ethylen aus der Trennzone antwortet.

**Revendications**

1. Procédé combiné de production d'éther et de production d'alcoylat, qui comprend les stades consistant à:

a) mettre en contact des oléfines comprenant au moins une oléfine tertiaire et un alcool pour former un premier mélange réactionnel comprenant l'éther, l'alcool et une partie de l'oléfine;

b) récupérer l'éther de ce premier mélange réactionnel en tant que premier produit du procédé combiné;

c) accumuler un liquide dans un récipient pour son utilisation ultérieure dans le stade de mise en contact a);

d) séparer un courant contenant des oléfines de ce premier mélange réactionnel;

e) mettre en contact au moins une partie de ce courant contenant des oléfines avec au moins une isoparaffine, en particulier une isoparaffine en $C_4$ à $C_6$, en présence d'un catalyseur d'alcoylation à l'acide fluorhydrique dans des conditions d'alcoylation à l'acide fluorhydrique pour former un second mélange réactionnel comprenant un alcoylat; et

f) à récupérer l'alcoylat de ce second mélange réactionnel en tant que produit supplémentaire du procédé sous la forme d'un courant de queues provenant d'une zone de fractionnement dans laquelle au moins une partie du second mélange réactionnel est fractionnée, et laquelle zone de fractionnement est munie d'un accumulateur de têtes dans lequel les têtes refroidies, condensées sortant de cette zone de fractionnement sont accumulées; caractérisé en ce qu'on distille un courant d'alimentation d'un mélange contenant un alcool et au moins un des gaz oxygène, azote, méthane, gaz combustible, éthane, hydrogène et éthylène dans une première zone de distillation, formant ainsi une première tête de distillation contenant une première partie de cet alcool et cet au moins un des gaz oxygène, azote, méthane, gaz combustible, éthane, hydrogène et éthylène et une première queue de distillation contenant une seconde partie de cet alcool; en ce qu'on condense au moins une partie de ces premières têtes de distillation contenant cet alcool et de cet au moins un des gaz oxygène, azote, méthane, gaz combustible, éthane, hydrogène et éthylène; puis en ce qu'on sépare ces premières têtes de distillation dans une zone de séparation en un alcool liquide séparé et au moins un des gaz oxygène, azote, méthane, gaz combustible, éthane, hydrogène et éthylène sous forme gazeuse; et en ce qu'on recueille ces premières têtes de distillation et en ce qu'on envoie ces premières têtes de distillation ainsi récupérées pour l'utilisation dans le stade de mise en contact a).

2. Procédé suivant la Revendication 1, caractérisé en ce qu'au moins une partie de cet alcool liquide séparé est introduite dans cette première zone de distillation en tant que partie de ce courant d'alimentation avec ce mélange contenant un alcool et au moins un des gaz oxygène, azote, méthane, gaz combustible, éthane, hydrogène et éthylène.

3. Procédé suivant les Revendications 1 ou 2, caractérisé en outre en ce qu'il comprend:

la séparation d'un liquide contenant de l'eau et de l'alcool de ce premier mélange réactionnel;

la distillation de ce liquide ainsi séparé contenant de l'eau et un alcool dans une seconde zone de distillation, formant ainsi des secondes têtes de distillation contenant cet alcool et une seconde queue de distillation contenant cette eau; et

la récupération de ces secondes têtes de distillation contenant cet alcool.

4. Procédé de la Revendication 3 caractérisé en ce qu'au moins une partie de ces secondes têtes de distillation récupérées est condensée en un liquide et en ce qu'au moins une partie du liquide ainsi condensé est introduite dans cette seconde zone de distillation en tant que courant de reflux.

9

5. Procédé de la Revendication 3, caractérisé en ce qu'au moins une partie de ces secondes têtes de distillation recueillies est condensée sous forme liquide et en ce qu'au moins une partie du liquide ainsi condensé est introduite dans cette première zone de distillation en tant que partie de ce courant d'alimentation avec ce mélange contenant un alcool et au moins un des gaz oxygène, azote, méthane, gaz combustible, éthane, hydrogène et éthylène.

6. Procédé de la Revendication 5, caractérisé en ce qu'au moins une partie du liquide ainsi condensé est introduite dans cette seconde zone de distillation en tant que courant de reflux.

7. Procédé de la Revendication 1, caractérisé en ce qu'au moins une partie de cet alcool liquide séparé est introduite dans cette première zone de distillation en tant que courant de reflux.

8. Procédé de la Revendication 1, caractérisé en ce que de 5 à 30% en volume de ce courant d'alimentation d'un mélange contenant un alcool et au moins un des gaz oxygène, azote, méthane, gaz combustible, éthane, hydrogène et éthylène sont transformés en ces premières têtes de distillation dans ce stade de distillation.

9. Procédé suivant l'une quelconque des Revendications 1 à 8, caractérisé en ce que cet alcool est le méthanol.

10. Procédé suivant l'une quelconque des Revendications 1 à 9, caractérisé en ce que cet au moins un des gaz oxygène, azote, méthane, gaz combustible, éthane, hydrogène et éthylène est brûlé et évacué dans l'atmosphère.

11. Système pour contrôler le procédé suivant l'une quelconque des Revendications 1 à 10, caractérisé par des moyens réagissant au débit de ce courant d'alimentation dans cette première zone de distillation pour contrôler l'apport de chaleur dans cette première zone de distillation; ou des moyens répondant au débit de ce courant d'alimentation dans cette première zone de distillation pour contrôler l'apport de chaleur dans cette première zone de distillation; ou des moyens réagissant au débit de ce courant d'alimentation dans cette première zone de distillation pour contrôler l'apport de chaleur dans cette première zone de distillation, et des moyens réagissant au niveau de ces premières queues de distillation dans cette première zone de distillation pour contrôler la production de ces premières queues de distillation en provenance de cette première zone de distillation; en particulier caractérisé en ce qu'il comprend en outre des moyens réagissant au niveau de l'alcool liquide séparé dans cette zone de séparation pour contrôler la production d'alcool liquide séparé provenant de cette zone de séparation dans cette première zone de distillation en tant que partie de ce courant d'alimentation.

12. Système pour contrôler le procédé suivant l'une quelconque des Revendications 1 à 10, caractérisé par des moyens réagissant au niveau d'alcool liquide séparé dans cette zone de séparation pour contrôler l'apport de chaleur dans cette première zone de distillation; ou des moyens réagissant au débit de ce courant d'alimentation dans cette première zone de distillation pour contrôler l'introduction de ce courant de reflux dans cette première zone de distillation; ou des moyens réagissant au niveau de ces premières queues de distillation dans cette première zone de distillation pour contrôler la production de ces premières queues de distillation provenant de cette première zone de distillation; ou des moyens réagissant à la température dans cette zone de séparation pour contrôler la condensation d'au moins une partie de ces premières têtes de distillation; ou des moyens répondant à la pression dans cette zone de séparation pour contrôler l'évacuation de cet au moins un gaz oxygène, azote, méthane, gaz combustible, éthane, hydrogène et éthylène de cette zone de séparation.

FIG. 2

FIG. 1A

0 103 870

FIG. 1B